(19) Europäisches Patentamt
European Patent Office
Office européen des brevets

(11) Veröffentlichungsnummer: **0 399 228 A1**

(12) **EUROPÄISCHE PATENTANMELDUNG**

(21) Anmeldenummer: **90107815.4**

(51) Int. Cl.5: **C07D 499/88**

(22) Anmeldetag: **25.04.90**

(30) Priorität: **29.04.89 DE 3914389**
**27.05.89 DE 3917287**

(43) Veröffentlichungstag der Anmeldung:
**28.11.90 Patentblatt 90/48**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Budt, Karl-Heinz, Dr.**
**Am Flachsland 18**
**D-6233 Kelkheim/Taunus(DE)**
Erfinder: **Dürckheimer, Walter, Dr.**
**Im Lerchenfeld 45**
**D-6234 Hattersheim am Main(DE)**
Erfinder: **Fischer, Gerd, Dr.**
**Johannesallee 6**
**D-6230 Frankfurt am Main(DE)**
Erfinder: **Hörlein, Rolf, Dr.**
**Assmannshäuser Weg 21**
**D-6000 Frankfurt am Main(DE)**
Erfinder: **Kirrstetter, Reiner, Dr.**
**Altenhainer Strasse 8a**
**D-6233 Kelkheim/Taunus(DE)**
Erfinder: **Lattrell, Rudolf, Dr.**
**Heuhohlweg 6h**
**D-6240 Königstein/Taunus(DE)**

(54) **Verfahren zur Herstellung von Penemverbindungen.**

(57) Verbindung I

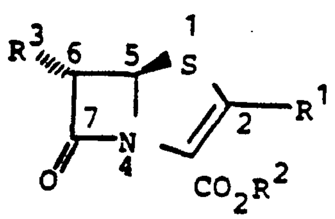

I

erhält man durch Umsetzung von Verbindung II

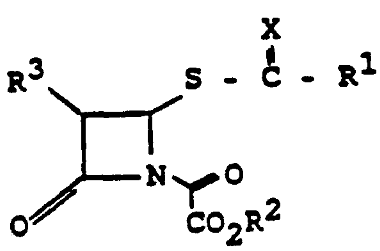

II,

mit Verbindung III

EP 0 399 228 A1

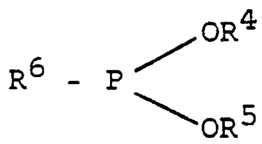

III,

## Verfahren zur Herstellung von Penemverbindungen

Die Erfindung betrifft ein Verfahren zur Herstellung von Penemverbindungen.

Penemderivate der Formel I sind wertvolle Verbindungen mit antibiotischen Eigenschaften. In einem literaturbekannten Syntheseverfahren für Penemderivate I werden Azetidinonderivate der Formel II mit Trialkylphosphiten intramolekular cyclisiert. Dieses Verfahren liefert jedoch oft nur schlechte Ausbeuten. Überdies ist die Reaktion langsam und erfordert erhöhte Reaktionstemperaturen, z.B. Rückfluß in Toluol. Unter diesen Reaktionsbedingungen tritt häufig eine teilweise Konfigurationsumkehr an C-5 ein, die zu unerwünschten (5S,6S)-Penemen führt.

Es wurde nun gefunden, daß sich die beschriebenen Nachteile des bekannten Verfahrens vermeiden lassen, wenn für die Cyclisierungsreaktion Alkylphosphonigsäuredialkylester anstelle von Trialkylphosphiten verwendet werden. Diese neuartigen Reagentien erlauben niedrigere Reaktionstemperaturen, z.B. Zimmertemperatur, und führen zu kürzeren Reaktionszeiten. Dadurch werden höhere Ausbeuten und reinere Produkte erzielt. Außerdem wird die unerwünschte Isomerisierung zu (5S,6S)-Penemen vermieden. Die als Nebenprodukte gebildeten
Alkylphosphonsäuredialkylester und
Alkylthiophosphonsäuredialkylester können auf einfache Weise abgetrennt werden.

Die Erfindung betrifft daher ein Verfahren zur Herstellung von Penemderivaten der Formel I, in denen die bevorzugte Konfiguration (5R,6S) ist

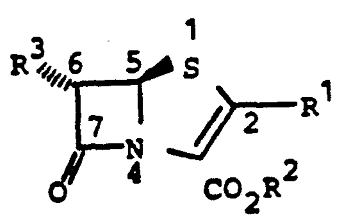

I

und worin

$R^1$ Wasserstoff, $(C_1\text{-}C_4)$-Alkyl, $(C_1\text{-}C_{12})$-Alkoxy, $(C_1\text{-}C_{12})$-Alkylthio, Phenoxy, Phenyl (wobei die Phenylringe unsubstituiert oder ein- oder zweifach substituiert sind durch Carboxy, $(C_1\text{-}C_4)$-Alkoxycarbonyl, Allyloxycarbonyl, Aminocarbonyl, $(C_1\text{-}C_4)$-Alkylaminocarbonyl, Cyano, F, Cl, Br), $(C_3\text{-}C_6)$-Cycloalkyl, $(C_3\text{-}C_6)$-Cycloalkyloxy, $(C_5\text{-}C_6)$-Oxacycloalkyl (gesättigt, ein- oder zweifach ungesättigt), $(C_3\text{-}C_6)$-Oxocycloalkyl, $(C_3\text{-}C_6)$-[1,1-bis-$(C_1\text{-}C_3)$-Alkyloxy]-cycloalkyl, $(C_3\text{-}C_6)$-[$(C_1\text{-}C_3)$-Alkylimino]-cycloalkyl, $(C_3\text{-}C_6)$-[Arylimino]cycloalkyl, $(C_3\text{-}C_6)$-Hydroxyiminocycloalkyl, $(C_3\text{-}C_6)$-$(C_1\text{-}C_3$-Alkyloxyimino)-cycloalkyl, in denen der Cycloalkylrest unsubstituiert oder ein- oder zweifach durch $C_1\text{-}C_3$-Alkyl, vorzugsweise Methyl, durch $(C_1\text{-}C_3)$-Alkoxy, vorzugsweise Methoxy, durch Halogen, vorzugsweise Chlor, oder durch Methylen substituiert ist und gesättigt ist oder eine oder zwei Doppelbindungen enthalten kann,

$R^2$ Wasserstoff oder eine übliche Carboxylschutzgruppe, die durch Hydrolyse, Photolyse, Oxidation, Reduktion oder enzymatisch abgespalten werden kann,

$R^3$ Wasserstoff, $(C_1\text{-}C_4)$-Alkyl, $(C_1\text{-}C_4)$-Alkenyl, $(C_1\text{-}C_4)$-Alkoxy, $(C_4\text{-}C_7)$-Cycloalkyl, Phenyl, 2-Oxo-1,3-dioxolyl, Triazolyl, Thiazolyl, Amino, Acylamino oder Alkoxy

bedeutet.

Als besonders bevorzugt kommen die folgenden Substituenten in Betracht: $R^1$ Wasserstoff, $(C_1\text{-}C_4)$-Alkyl (z.B. Methyl, Ethyl, Hydroxymethyl und Aminomethyl), $(C_1\text{-}C_4)$-Alkoxy (z.B. Methoxy und Ethoxy), $(C_1\text{-}C_3)$-Alkylthio (z.B. Methylthio, Ethylthio und Propylthio), Phenoxy (z.B. 4-Carboxamidophenoxy oder 4-Cyanophenoxy), Phenyl (z.B. 4-Carboxamidophenyl oder 4-Cyanophenyl), gesättigtes oder ungesättigten $(C_5\text{-}C_6)$-Oxacycloalkyl (z.B. Tetrahydrofuryl oder Furyl), $(C_4\text{-}C_6)$-Oxocycloalkyl (z.B. 1-Oxo-cyclobut-3-yl), 3-Hydroxyiminocyclobutyl, 3-Methoxyiminocyclobutyl und 3,3-Dimethoxycyclobutyl.

$R^3$ 1-Hydroxyethyl (worin die OH-Gruppe frei ist oder geschützt durch Trimethylsilyl, Diphenyl-tert.-butylsilyl, Allyloxycarbonyl, Trichlorethyloxycarbonyl oder 4-Nitrobenzyloxycarbonyl), $(C_1\text{-}C_3)$-Alkoxy (z.B. Methoxy oder Ethoxy), $(C_1\text{-}C_3)$-Alkenyl, Triazolyl oder Thiazolyl.

Die $(C_1\text{-}C_4)$-Alkyl- und $(C_1\text{-}C_4)$-Alkoxy-Gruppen in dem Substituenten $R^1$ sind entweder unsubstituiert oder ein-oder zweifach substituiert durch Hydroxy, $(C_1\text{-}C_4)$-Alkoxy, $(C_1\text{-}C_4)$-Acyloxy, Amino, $(C_1\text{-}C_4)$-Alkylamino, $(C_1\text{-}C_4)$-Acylamino, Mercapto, $(C_1\text{-}C_4)$-Alkylthio oder Heterocyclylthio, z.B Thiazolyl-, Thiadiazolyl-,

Pyridylthio.

Phenylkerne sind ebenfalls unsubstituiert oder ein- oder zweifach substituiert durch Carboxy, $(C_1-C_4)$-Alkoxycarbonyl, Allyloxycarbonyl, Aminocarbonyl, $(C_1-C_4)$-Alkylaminocarbonyl, Cyano oder Halogen, vorzugsweise F, Cl, Br.

Die $(C_1-C_4)$-Alkyl- und $(C_1-C_4)$-Alkoxy-Gruppen in $R^3$ sind entweder unsubstituiert oder substituiert durch Hydroxy, $(C_1-C_4)$-Alkoxy, $(C_1-C_4)$-Acyloxy, Amino, $(C_1-C_4)$-Alkylamino, $(C_1-C_4)$-Acylamino, Mercapto, $(C_1-C_4)$-Alkylthio oder Heterocyclylthio, wobei eine OH-Gruppe frei sein kann oder geschützt durch Trimethylsilyl, Diphenyl-tert.-butylsilyl, Allyloxycarbonyl, Trichlorethoxycarbonyl oder 4-Nitrobenzyloxycarbonyl.

Nach dem erfindungsgemäßen Verfahren werden die Verbindungen der Formel I durch Umsetzen einer Verbindung der Formel II

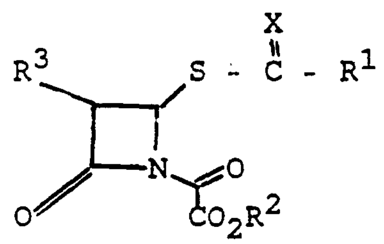

II,

worin

X Sauerstoff oder Schwefel bedeutet und

$R^1$, $R^2$ und $R^3$ die obige Bedeutung haben, mit einer dreiwertigen organischen Phosphorverbindung der Formel III

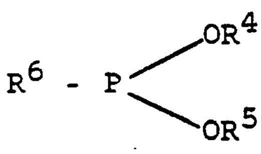

III,

hergestellt,

worin

$R^6$ $(C_1-C_4)$-Alkyl, beispielsweise Methyl, Ethyl oder Trifluormethyl, Phenyl, das substituiert sein kann durch $(C_1-C_3)$-Alkyl oder $(C_1-C_3)$-Alkoxy, und

$R^4$ und $R^5$ gleich oder verschieden sind und $(C_1-C_4)$-Alkyl, Allyl, Benzyl oder Phenyl, das substituiert sein kann durch $(C_1-C_3)$-Alkyl oder $(C_1-C_3)$-Alkoxy bedeuten.

Die Reaktion zwischen einer Verbindung II und einer Verbindung III kann in einem geeigneten organischen Lösungsmittel durchgeführt werden, beispielsweise in Tetrahydrofuran, Ethylacetat, einem aromatischen Kohlenwasserstoff wie Benzol, Toluol oder Xylol, oder einem halogenierten Kohlenwasserstoff, wie Dichlormethan, Trichlormethan oder 1,1,2-Trichlorethan.

Die Reaktionstemperatur kann zwischen +10° C und 160° C, vorzugsweise zwischen +20° C und +70° C variieren.

Die Konzentration der zu cyclisierenden Verbindung II beträgt zwischen 1 mmol/1 und 100 mmol/1, vorzugsweise zwischen 2 mmol/1 und 20 mmol/1.

Die Menge der Verbindung III kann zwischen 2 und 8 Moläquivalenten, vorzugsweise zwischen 2 und 6 Moläquivalenten bezogen auf II betragen.

Die Verbindungen der Formeln II und III sind bekannt oder können gemäß literaturbekannten Verfahren hergestellt werden.

Die folgenden Beispiele dienen zur weiteren Erläuterung der Erfindung.

**Beispiel 1**

(5R,6S)-6-[(1R)-tert.-Butyldimethylsilyloxyethyl]-2-(4-aminocarbonylphenoxy)penem-3-carbonsäure-(4-nitrobenzyl)ester

130 mg (0,2 mmol) (3S,4R)-4-[(Aminocarbonyl)phenoxythiocarbonylthio]-3-[(1R)-tert.-butyldimethylsilyloxyethyl)]-1-(4-Nitrobenzyloxycarbonyl)-azetidin-2-on wurden bei 55 °C in 90 ml $CHCl_3$ unter Argonatmosphäre gelöst. Dazu gab man binnen 30 Minuten eine Lösung von 90 mg (0,8 mmol) Methylphosphonigsäuredimethylester $CH_3P(OCH_3)_2$ in 10 ml $CHCl_3$, rührte nach Beendigung der Zugabe noch 2 Stunden und arbeitete das abgekühlte Gemisch auf. Man extrahierte mit 1 N $NaHCO_3$-Lösung, 1 N $KHSO_4$-Lösung und Wasser, trocknete mit Magnesiumsulfat und engte die organische Phase im Vakuum ein. Das Rohprodukt ergab nach Flash-Chromatographie ($SiO_2$ 70-200 µm + 10 % $H_2O$; zuerst Toluol + Ethylacetat 20 + 1, dann 1 + 1 zur Elution) die Titelverbindung in 83 % Ausbeute.

Analog wurden die in der Tabelle 1 aufgeführten Verbindungen I aus den in Tabelle 2 aufgeführten Ausgangssubstanzen II unter den in Tabelle 3 aufgeführten Reaktionsbedingungen erhalten.

**Tabelle 1**

NMR-Daten der erfindungsgemäßen Peneme I

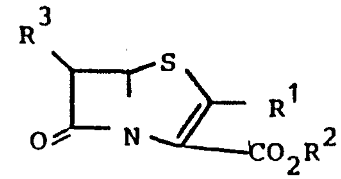

| Beispiel | $R^1$ | $R^2$ | $R^3$ | $^1H$-NMR ($CDCl_3$) : δ (ppm) |
|---|---|---|---|---|
| 1 | 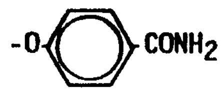 | PNB | $-CH(OTBDMS)CH_3$ | 8.17, 7.55 (4H, AA'BB', J=8.7 Hz, aromat. H von PNB); 7.83, 7.21 (4H AA'BB', J=8.8 aromat. H von Benzamid); 5,65 (1H, d, J=1.4, H-5); 5.38, 5.21 (2H, AB, J=13.8, Benzyl-H); 4.41-4,42 (1H, m, -CH(OTBDMS)-); 3.76 (1H, dd J=1.4, 4.8, H-6); 1.25 (3H, d, J=6.2, $CH_3$-); 0.82 (9H, s. tert.-Butyl-H); 0.09, 0.05 (6H, 2 x s,. $Si(CH_3)_2$). |
| 2 | 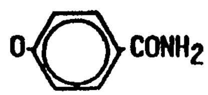 | $-CH_2CH=CH_2$ | " | 7.83, 7.21 (4H, AA'BB', J=8.8, aromat. H von Benzamid); 5.78-5.94 (1H, m, -CH=); 5.62 (1H, d, J=1.4, H-5); 5.40-5.17 (2H, m, $=CH_2$); 4.66 (2H, m, $CO_2$-$CH_2$); 4.25 (1H, m, -CH(OTBDMS)-); 3.71 (1H, dd, J=1.4, 4.8, H-6); 1.25 (3H, d, J=6.2, $CH_3$-); 0.88 (9H, s, tert.-Butyl-H); 0.09, 0.05 (6H, 2 x s, $Si(CH_3)_2$). |
| 3 | 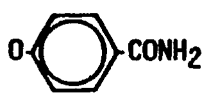 | $-CH_2CH_2SiMe_3$ | " | 7.82, 7.20 (4H, AA'BB', J=8.8, aromat. H von Benzamid); 5.61 (1H, d, J=1.4, H-5); 4.20-4.30 (3H, m, -CH(OTBDMS)- und $CO_2CH_2$); 3.72 (1H, dd, J=1.4, 4.8, H-6); 1.25 (3H, d, J=6.2, $CH_3$-); 0.96 (2H, m, $CH_2Si$); 0.87 (9H, s, tert.-Butyl-H); 0.1-0.01 (zus. 15H, 3 x s, $Si(CH_3)_3$). |

* TBDMS = t-butyldimethylsilyl  Me = Methyl  PNB = para-Nitrobenzyl  TCE = 1,1,2-Trichlorethan

**Fortsetzung Tabelle 1**

| Beispiel | $R^1$ | $R^2$ | $R^3$ | $^1H$-NMR ($CDCl_3$) : δ (ppm) |
|---|---|---|---|---|
| 4 | $OCH_3$ | $-CH_2CH=CH_2$ | $-CH(OTBDMS)CH_3$ | 5.85-6.02 (1H, m, -CH=); 5.55 (1H, d, J=1.4, H-5); 5.43-5.17 (zus. 2H, m, $=CH_2$); 4.65 (2H, m, $CO_2$-$CH_2$); 4.25 (1H, m, -CH(OTBDMS)-); 4.00 (3H, s, $OCH_3$); 3.65 (1H, dd, J=1.4, 4.8, H-6); 1,25 (3H, d, J=6.2, $CH_3$-); 0.90 (9H, s, tert-Butyl-H); 0.10 (6H, 2 x s, $Si(CH_3)_2$). |
| 5 | $OCH_3$ | -PNB | " | 8.20, 7.60 (4H,AA'BB, J=8.7 Hz, aromat. H von PNB); 5.61 (1H, d, J=1.4, H-5); 5.39 und 5.19 (2H, ABq, 14Hz, Benzyl-H); 4.25 (1H, m, -CH(OTBDMS)-); 4.04 (3H, s, $OCH_3$); 3.71 (1H, dd, J=1.4, 4.8, H-6); 1.27 (3H, d, J=6.2), $CH_3$-); 0.82 (9H, s, tert.-Butyl-H); 0.09, 0.05 (6H, 2 x s, $Si(CH_3)_2$). |
| 6 | $OCH_3$ | $-CH_2CH_2SiMe_3$ | " | 5.52 (1H, d. J=1.4, H-5); 4.25 (zus. 3H, m, -CH(OTDBMS)-, $CH_2$-OCO); 4.00 (3H, s, $OCH_3$), 3.65 (1H, dd, J=1.4, 4.8, H-6); 1.25 (3H, d, J=6.2, $CH_3$-); 1.05 (2H, m, $CH_2Si$); 0.90 (9H, s, tert.-Butyl-H); 0.5-0.1 (15H, 3 x s, $Si(CH_3)_3$). |
| 7 | O- 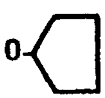 | -PNB | " | 8.29, 7.59 (4H, AA'BB', J=8.7 Hz, aromat H von PNB); 5.55 (1H, d, J=1.4, H-5); 5.39 und 5.19 (2H, ABq, 14Hz, Benzyl-H); 4.72 (1H, m, 1'-CH); 4.25 (1H, m, -CH(OTBDMS)-); 3.66 (1H, dd, J=1.4, 4.8, H-6); 2.0-1.5 (zus. 8H, m, Cyclopentyl-$CH_2$); 1.27 (3H, d, J=6.2, $CH_3$-); 0.82 (9H, s, tert.-Butyl-H); 0.08, 0.05 (6H, 2 x s, $Si(CH_3)_2$). |

**Fortsetzung Tabelle 1**

| Beispiel | $R^1$ | $R^2$ | $R^3$ | $^1H$-NMR ($CDCl_3$) : δ (ppm) |
|---|---|---|---|---|
| 8 | O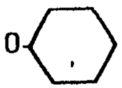 | -PNB | $-CH(OTBDMS)CH_3$ | 8.19, 7.61 (4H, AA'BB', J=8.7Hz, aromat. H von PNB); 5.55 (1H, d, J=1.4, H-5); 5.29 und 5.19 (2H, ABq, 14Hz, Benzyl-H); 4.21-4-12 (zus. 3H, m, 1'-CH und -CH(OTBDMS)-); 3.66 (1H, dd, J=1.4, 4.8, H-6); 2.05-1.3 (zus. 10H, m, Cyclohexyl-$CH_2$); 1.27 (3H, d, J=6.2, $CH_3$-); 0.82 (9H, s, tert.-Butyl-H); 0.08, 0.05 (6H, 2 x s, $Si(CH_3)_2$). |
| 9 | $SCH_3$ | $-CH_2CH=CH_2$ | " | 5.85-6.02 (1H, m, -CH=); 5.61 (1H, d, J=1.4, H-5); 5.43, 5.37, 5.22, 5.19 (zus. 2H, 4 x d, $=CH_2$); 4.70 (2H, m, $CO_2$-$CH_2$); 4.22 (1H, m, -CH(OTBDMS)-); 3.68 (1H, dd, J=1.4, 4.8, H-6); 2.51 (3H, s, $SCH_3$); 1.23 (3H, d, J=6.2, $CH_3$); 0.88 (9H, s, tert.-Butyl-H); 0.10 (6H, 2 x s, $Si(CH_3)_2$). |
| 10 | $SCH_3$ | -PNB | " | 8.21, 7.51 (4H, AA'BB', J=8.7 Hz, aromat. H von PNB); 5.66 (1H, d, J=1.4, H-5); 5.41 und 5.21 (2H, ABq, 14Hz, Benzyl-H); 4.25 (1H, m, -CH(OTBDMS)-); 3.71, 1H, dd, j=1.4, 4.8, H-6); 2.52 (3H, s, $SCH_3$); 1.25 (3H, d, J=6.2, $CH_3$-); 0.82 (9H, s, tert.-Butyl-H); 0.09, 0.05 (6H, 2 x s, $Si(CH_3)_2$). |
| 11 | $SCH_3$ | $-CH_2CH_2SiMe_3$ | " | 5.59 (1H, d, J=1.4, H-5); 4.0-4.31 (zus. 3H, m, -CH(OTBDMS)-, $CH_2$-OCO); 3.66 (1H, dd, J=1.4, 4.8, H-6); 2.50 (3H, s, $SCH_3$); 1.27 (3H, d, J=6.2, $CH_3$-); 1.08 (2H, m, $CH_2Si$); 0.89 (9H, s, tert.-Butyl-H); 0.01-0.11 (15H, 3 x s, $Si(CH_3)_2$). |

**Fortsetzung Tabelle 1**

| Beispiel | $R^1$ | $R^2$ | $R^3$ | $^1$H-NMR ($CDCl_3$) : δ (ppm) |
|---|---|---|---|---|
| 12 | 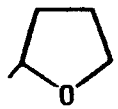 | $-CH_2CH_2SiMe_3$ | $-CH(OTBDMS)CH_3$ | 5.52 und 5.45 (zus. 1H, d, J=1.4, H-5); 5.42-5.35 (1H, m, 2'-CH); 4.31-4.15 (zus. 3H, m, -CH(OTBDMS)- und $CH_2$-OCO); 4.03-3.77 (2H, m, 5'-$CH_2$); 3.64 (1H, m, H-6); 2.51-2.34 (1H, m, 3',4'-$CH_2$); 2.05-1.71 (3H, m, 3',4'-$CH_2$); 1.25 (zus. 3H, m, $CH_3$-); 1.08 (2H, m, $CH_2Si$); 0.87 (9H, m, $CH_2Si$ und tert.-Butyl-H); 0.09-0.11 (15H, m, $Si(CH_3)_3$). |
| 13 | 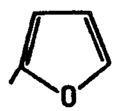 | $-CH_2CH_2SiMe_3$ | " | 7.68, 7.52, 6.53 (zus. 3H, 3 x m, Furyl-H); 5.56 (1H, d, J=1.4; H-5); 4.34-4.21 (zus. 3H, m, -CH(OTBDMS)- und $CH_2$-OCO); 3.69 (1H, dd, J=1.4, 4.8, H-6); 1.28 (3H, d, J=6.2, $CH_3$-); 1.10 (2H, m, $CH_2Si$); 0.91 (9H, m, tert.-Butyl-H); 0.01-0.11 (15H, 3 x s, $Si(CH_3)_3$). |
| 14<br>15 | 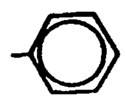 | $-CH_2CH_2SiMe_3$ | " | 7.50, 7.39 (zus. 5H, 2 x m, Phenyl-H); 5.69 (1H, d, J=1.4, H-5); 4.31 (1H, m, -CH(OTBDMS)-); 4.19 (2H, m, $CH_2$-OCO); 3.78 (1H, dd, J=1.4, 4.8, H-6); 1.32 (3H, d, J=6.2, $CH_3$-); 0.93 (zus. 11H, m, $CH_2Si$ und tert.-Butyl-H); 0.01-0.11 (15H, 3 x s, $Si(CH_3)_3$). |

* Die Verbindung "14" wird aus 2 verschiedenen Edukten in Beispiel 14 und 15 hergestellt

**Fortsetzung Tabelle 1**

| Beispiel | $R^1$ | $R^2$ | $R^3$ | $^1H$-NMR ($CDCl_3$) : δ (ppm) |
|---|---|---|---|---|
| 16 | 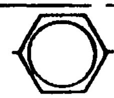 $-CO_2CH_2CH=CH_2$ | $-CH_2CH_2SiMe_3$ | $-CH(OTBDMS)CH_3$ | 8.06, 7.53 (4H, AA'BB, J=8.8 Hz, aromat. H); 6.1-5.94 (1H, m, -CH=); 5.71 (1H, d, J=1.4, H-5); 5.44-5.20 (2H, m, $=CH_2$); 4.84 (2H, m, $CO_2$-$CH_2$/Allyl); 4.27 (1H, m, -CH(OTBDMS)-); 4.16 (2H, m, $CO_2CH_2$/TMSE); 3.78 (1H, dd, J=1.4, 4.8, H-6); 1.28 (3H, d, J=6.2, $CH_3$-); 0.91 (9H, s, tert.-Butyl-H); 0.11-0.01 (15H, 4 x s, $Si(CH_3)_2$). |
| 17 | 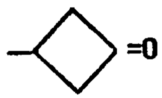 | $-CH_2CH_2SiMe_3$ | " | 5.58 (d, J=1,4 Hz, H-5); 4.52 (m, 1H, Cyclobutyl); 4.25 (m, 3H, $CO_2CH_2$ und $CHCH_3$); 3.70 (dd, J=5 und 1Hz, H-6); 3.15-3.55 (m, 4 Cyclobutyl-H); 1.28 (3H, d, J=6Hz, $CHCH_3$); 1.08 (m, 2H, $CH_2Si$); 0.88 (s, 9H, tert.-Butyl-H); 0.18 (s, 6H, $Si(CH_3)_2$); 0.06 (s, 9H, $Si(CH_3)_3$) |
| 18 | 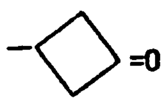 | $-CH_2CH=CH_2$ | " | 5.86-6.03 (m, 1=CH); 5.61 (d, 7=1Hz, H-5); 5.22-5.45 (m, $2=CH_2$); 4.72 (m, 2H, $CO_2CH_2$); 4.51 (m, 1H, Cyclobutyl); 4.25 (m, $CHCH_3$); 3.72 (dd, J=5 und 1 Hz, H-6); 3.15-3.55 (m, 4-Cyclobutyl-H); 1.28 (3H, d, J=6Hz, $CHCH_3$); 0.88 (s, 9H, tert.-Butyl-H); 0.10 (s, 6H, $Si(CH_3)_2$); |
| 19 | 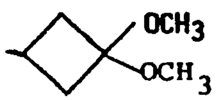 | $-CH_2CH_2SiMe_3$ | " | 5.52 (d, J=1Hz, H-5); 4.2-4.32 (m, 3H, $CO_2CH_2$ und $CHCH_3$); 4.0-4.14 (m, 1H, Cyclobutyl); 3.67 (dd, J=5 und 1Hz, H-6); 3.15 und 3.16 (je s, 2 x 3H, $OCH_3$); 2.5-2.7 (m, 2H, Cyclobutyl); 2.1-2.22 (m, 2H, Cyclobutyl), 1.28 (3H, d, J=6Hz, $CHCH_3$); 1.08 (m, 2H, $CH_2Si$); 0.88 (s, 9H, tert.-Butyl-H), 0.10 (s, 6H, $Si(CH_3)_2$); 0.06 (s, 9H, $Si(CH_3)_3$) |

**Tabelle 2**

Ausgangsverbindungen

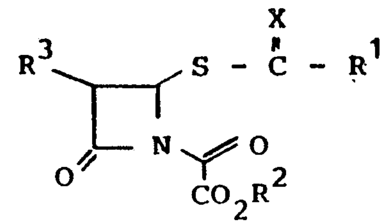

| Beispiel | $R^1$ | $R^2$ | $R^3$ | X |
|---|---|---|---|---|
| 1 | 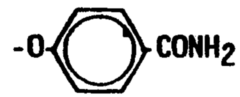 | -PNB | -CH(OTBDMS) | S |
| 2 | 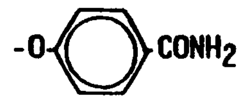 | $-CH_2CH=CH_2$ | " | S |
| 3 | 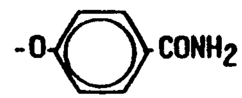 | $-CH_2CH_2SiMe_3$ | " | S |
| 4 | $OCH_3$ | $-CH_2CH=CH_2$ | " | S |
| 5 | $OCH_3$ | -PNB | " | S |
| 6 | $OCH_3$ | $-CH_2CH_2SiMe_3$ | " | S |
| 7 | 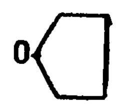 | -PNB | " | S |
| 8 | 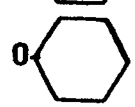 | -PNB | " | S |
| 9 | $SCH_3$ | $-CH_2CH=CH_2$ | " | S |

**Fortsetzung Tabelle 2**

| Beispiel | $R^1$ | $R^2$ | $R^3$ | X |
|---|---|---|---|---|
| 10 | $SCH_3$ | -PNB | " | S |
| 11 | $SCH_3$ | $-CH_2CH_2SiMe_3$ | " | S |
| 12 | 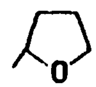 | $-CH_2CH_2SiMe_3$ | " | O |
| 13 | 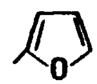 | $-CH_2CH_2SiMe_3$ | " | O |
| 14 | 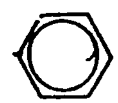 | $-CH_2CH_2SiMe_3$ | " | O |
| 15 | 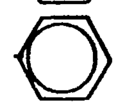 | $-CH_2CH_2SiMe_3$ | " | S |
| 16 | 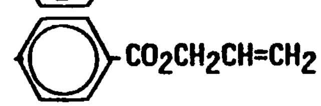 | $-CH_2CH_2SiMe_3$ | " | O |
| 17 | 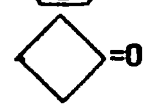 | $-CH_2CH_2SiMe_3$ | " | O |
| 18 | 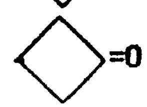 | $-CH_2CH=CH_2$ | " | O |
| 19 | 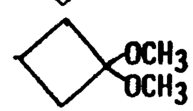 | $-CH_2CH_2SiMe_3$ | " | O |

**Tabelle 3**

Reaktionsbedingungen für die Cyclisierungsreaktion

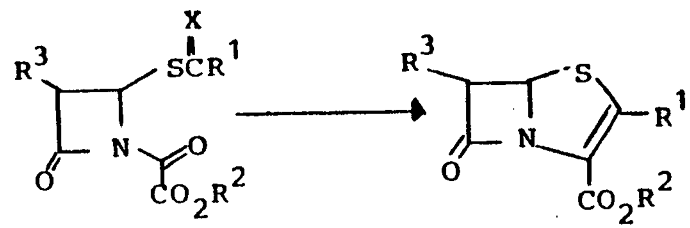

| Beisp. Nr. | Reagenz III | Äquivalente III | Konz. (mmol/l) | Lösungs-mittel | Temp. (°C) | Reaktions-zeit (Std.) | Ausbeute % |
|---|---|---|---|---|---|---|---|
| 1 | $CH_3P(OCH_3)_2$ | 4 | 20 | $CHCl_3$ | 55 | 2 | 83 |
| 2 | " | 3 | 20 | 1.1.2-TCE | 60 | 1 | 57 |
| 3 | " | 4 | 2 | $CHCl_3$ | " | 2 | 62 |
| 4 | $CH_3P(OC_2H_5)_2$ | 4 | 20 | " | " | 0,5 | 38 |
| 5 | " | 4 | 20 | " | " | 0,5 | 55 |
| 6 | " | 4 | 20 | " | " | 0,5 | 48 |
| 7 | " | 4 | 2 | " | " | 2 | 80 |
| 8 | " | 4 | 2 | " | " | 3 | 70 |
| 9 | " | 6 | 20 | " | 55 | 1 | 67 |
| 10 | " | 6 | 20 | " | " | 1 | 61 |
| 11 | " | 6 | 20 | " | " | 1 | 75 |
| 12 | " | 4 | 20 | " | 60 | 3 | 37 |
| 13 | " | 4 | 20 | " | " | 6 | 55 |
| 14 | " | 5 | 20 | " | 55 | 1 | 25 |
| 15 | $CH_3P(OCH_3)_2$ | 4 | 20 | " | 60 | 0,5 | 20 |
| 16 | $CH_3P(OC_2H_5)_2$ | 4 | 20 | " | " | 1 | 55 |
| 17 | " | 4 | 40 | 1.1.2-TCE | 70 | 2,5 | 72 |
| 18 | " | 4 | 40 | " | " | 2,5 | 58 |
| 19 | " | 4 | 40 | " | " | 2,5 | 42 |

**Ansprüche**

1. Verfahren zum Herstellen einer Penem-Verbindung I

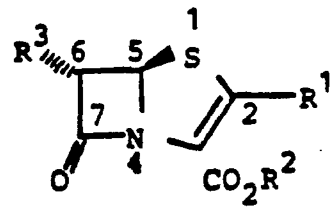

I

in welcher bedeuten:

$R^1$ Wasserstoff, $(C_1\text{-}C_4)$-Alkyl, $(C_1\text{-}C_{12})$-Alkoxy, $(C_1\text{-}C_{12})$-Alkylthio, Phenoxy, Phenyl (wobei die Phenylringe unsubstituiert oder ein- oder zweifach substituiert sind durch Carboxy, $(C_1\text{-}C_4)$-Alkoxycarbonyl, Allyloxycarbonyl, Aminocarbonyl, $(C_1\text{-}C_4)$-Alkylaminocarbonyl, Cyano, F, Cl, Br), $(C_3\text{-}C_6)$-Cycloalkyl, $(C_3\text{-}C_6)$-Cycloalkyloxy, $(C_5\text{-}C_6)$-Oxacycloalkyl (gesättigt, ein- oder zweifach ungesättigt), $(C_3\text{-}C_6)$-Oxocycloalkyl, $(C_3\text{-}C_6)$-[1,1-bis-$(C_1\text{-}C_3)$-Alkyloxy]-cycloalkyl, $(C_3\text{-}C_6)$-[$(C_1\text{-}C_3)$-Alkylimino]-cycloalkyl, $(C_3\text{-}C_6)$-Aryliminocycloalkyl, $(C_3\text{-}C_6)$-Hydroxyiminocycloalkyl, $(C_3\text{-}C_6)$-$(C_1\text{-}C_3$-Alkyloxyimino)-cycloalkyl, in denen der Cycloalkylrest unsubstituiert oder ein- oder zweifach durch $C_1\text{-}C_3$-Alkyl, durch $(C_1\text{-}C_3)$-Alkoxy, durch Halogen oder durch Methylen substituiert ist und gesättigt ist oder eine oder zwei Doppelbindungen enthalten kann,

$R^2$ Wasserstoff oder eine übliche Carboxylschutzgruppe, die durch Hydrolyse, Photolyse, Oxidation, Reduktion oder enzymatisch abgespalten werden kann, $R^3$ Wasserstoff, $(C_1\text{-}C_4)$-Alkyl, $(C_1\text{-}C_4)$-Alkenyl, $(C_1\text{-}C_4)$-Alkoxy, $(C_4\text{-}C_7)$-Cycloalkyl, Phenyl, 2-Oxo-1,3-dioxolyl, Triazolyl, Thiazolyl, Amino, Acylamino oder Alkoxy

dadurch gekennzeichnet, daß man eine Verbindung der Formel II

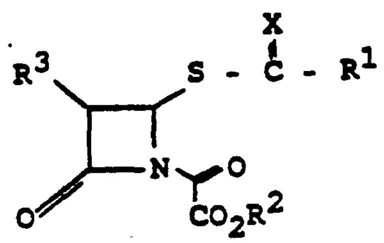

II,

worin

X Sauerstoff oder Schwefel bedeutet und

$R^1$, $R^2$ und $R^3$ die obige Bedeutung haben, mit einer dreiwertigen organischen Phosphorverbindung der Formel III

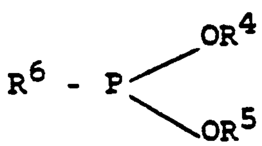

III,

umsetzt,

worin

$R^6$ $(C_1\text{-}C_4)$-Alkyl, Phenyl, das substituiert sein kann durch $(C_1\text{-}C_3)$-Alkyl oder $(C_1\text{-}C_3)$Alkoxy, und

$R^4$ und $R^5$ gleich oder verschieden sind und $(C_1\text{-}C_4)$-Alkyl, Allyl, Benzyl oder Phenyl, das substituiert sein kann durch $(C_1\text{-}C_3)$-Alkyl oder $(C_1\text{-}C_3)$Alkoxy

bedeuten.

2. Verfahren nach Anspruch 1, dadurch gekennzeichnet, daß man die Umsetzung von Verbindung II mit III in einem organischen Lösungsmittel durchführt.

3. Verfahren nach Anspruch 1, dadurch gekennzeichnet, daß man die Reaktion zwischen $+10\,^\circ C$ und $+160\,^\circ C$ durchführt.

4. Verfahren nach Anspruch 1, dadurch gekennzeichnet, daß

$R^1$ Wasserstoff, $(C_1\text{-}C_4)$-Alkyl, $(C_1\text{-}C_4)$-Alkoxy, $(C_1\text{-}C_3)$-Alkylthio, Phenoxy, Phenyl (wobei die Phenylkerne durch $(C_1\text{-}C_4)$-Alkoxycarbonyl, Aminocarbonyl oder Cyano substituiert wird), $(C_5\text{-}C_6)$-Oxacycloalkyl, $(C_4\text{-}C_6)$-Oxocycloalkyl, 3-Hydroxyiminocyclobutyl, 3-Methoxyiminocyclobutyl, 3,3-Dimethoxycyclobutyl, 3-Methoxy-2-cyclobuten-1-yl, 2-Methoxy-1-cyclobuten-1-yl und 4-Methoxy-3-cyclohexen-1-yl
$R^3$ 1-Hydroxyethyl (worin die OH-Gruppe frei ist oder geschützt durch Trimethylsilyl, Diphenyl-tert.-butylsilyl, Allyloxycarbonyl, Trichlorethoxycarbonyl oder 4-Nitrobenzyloxycarbonyl), ($C_1$-$C_3$)-Alkoxy, ($C_1$-$C_3$)-Alkenyl
bedeuten.

Europäisches Patentamt

# EUROPÄISCHER RECHERCHENBERICHT

Nummer der Anmeldung

EP 90 10 7815

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 429 102 (FARMITALIA CARLO ERBA S.p.A.)<br>* Ansprüche; Seite 15 *<br>--- | 1-4 | C 07 D 499/88 |
| A | EP-A-0 275 002 (HOECHST)<br>* Ansprüche *<br>----- | 1-4 | |
| | | | RECHERCHIERTE SACHGEBIETE (Int. Cl.5) |
| | | | C 07 D 499/00 |

Der vorliegende Recherchenbericht wurde für alle Patentansprüche erstellt

| Recherchenort | Abschlußdatum der Recherche | Prüfer |
|---|---|---|
| DEN HAAG | 14-09-1990 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument